# EUROPEAN PATENT APPLICATION

(11) **EP 4 309 659 A1**
(43) Date of publication of application: **24.01.2024**
(21) Application number: 22770464.0
(22) Date of filing: 14.03.2022
(51) Int. Cl.: A61K 31/711, A61K 31/7105, A61P 27/06, A61P 25/02

(54) **COMPLEX FOR TREATING OPTIC NERVE DISEASE, AND PREPARATION METHOD THEREFOR AND USE THEREOF**

(30) Priority: 18.03.2021 CN 202110293024
(71) Applicant: Chengdu Genreze Gene Technology Co., Ltd, Chengdu, Sichuan 610097 (CN)
(72) Inventor: LIN, Yunfeng, Chengdu, Sichuan 610044 (CN); LI, Jiajie, Chengdu, Sichuan 610044 (CN); CAI, Xiaoxiao, Chengdu, Sichuan 610044 (CN)
(74) Representative: Plougmann Vingtoft a/s
(86) International application number: PCT/CN2022/080771
(87) International publication number: WO 2022/194109

(57) **Abstract**

Disclosed is a complex tFNA-miR22 for treating optic nerve disease, which complex is composed of tetrahedral DNA and miR-22 according to a molar ratio of 1: (1-4). The tFNA-miR22 can effectively inhibit apoptosis of retinal ganglion cells and promote the release of a brain-derived neurotrophic factor (BDNF), thereby achieving a good protection effect for the retinal ganglion cells. The tFNA-miR22 is used for preparing optic nerve protection drugs, the treatment of neurodegenerative optic nerve diseases including glaucoma is facilitated, and the tFNA-miR22 has very good application prospects.

## Description

### Field of the Invention

The invention belongs to the field of biomedicine, and in particular relates to a complex for the treatment of optic nerve diseases and its preparation method and use.

### Background of the Invention

Glaucoma is a group of diseases that threaten and injure the optic nerve and its visual pathways and thus lead to visual dysfunction, and is the leading cause of irreversible blindness worldwide. Primary open-angle glaucoma is a specific type of optic nerve disease characterized by progressive injury to the retinal ganglion cells (RGCs) and their axons, with characteristic optic nerve atrophy and visual field defects. Glaucoma tends to be insidious and progresses slowly, with no obvious symptoms in the early stages and the visual field gradually shrinking until blindness. There are nearly 21 million glaucoma patients in China, which will likely produce nearly 6.3 million blind people and over 10 million visually impaired people.

Currently, the main treatment for glaucoma is to reduce intraocular pressure (IOP) medically or surgically to slow down the injure to the optic nerve.. However, lowering intraocular pressure alone cannot completely and effectively prevent or reverse optic nerve injury caused by retinal ganglion cell death. In some glaucoma patients, the injury of retinal ganglion cells continues to progress even after the intraocular pressure is controlled, and vision may be completely lost if no effective treatment is taken.

Optic nerve protection has been a frontier hot spot in ophthalmology research in recent years, as well as a difficult point in the treatment of glaucoma. At present, the commonly used drugs in clinical practice, such as prostaglandins, β-receptor blockers, adrenergic agonists, carbonic anhydrase inhibitors, and myotic agents including pilocarpine are all ocular hypotensive agents , while neuroprotective drugs are scarce.

Tetrahedral DNA (TDN), also known as tetrahedral framework nuclear acids (tFNAs) and tetrahedral DNA nanostructures, is a tetrahedral nanostructure formed by 4 single-stranded DNAs through denaturation and renaturation, with complementary base pairing between the strands. It is easy to synthesize and has high biocompatibility, and is usually used as a carrier for certain drugs. Chinese patent application CN109806275A discloses the use of tetrahedral DNA to promote the proliferation, differentiation and/or migration of neural stem cells, but does not disclose the effect of tetrahedral DNA on the protection of optic nerve.

CN112007044A discloses the use of tetrahedral DNA for the preparation of drugs to prevent oxidative stress in retinal ganglion cells, showing that tetrahedral DNA alone or its complex with miR-155 can be used for the treatment of wet macular degeneration (AMD).

As one of the most frequently studied microRNAs, miR-22 is involved in a variety of biological processes such as cardiac remodeling, cell cycle regulation, proliferation, and differentiation, and has various anti-neurodegenerative and anti-tumor effects, including inhibition of neuronal apoptosis and participation in the regulation of brain-derived neurophilic factor (BDNF)-related signaling pathways, and inhibition of proliferation, invasion, and migration of a variety of tumor cells. Romano et al. revealed that miR-22 is a target gene for predicting glaucoma, but the use of miR-22 as a target gene for treating glaucoma has not been disclosed (Romano GL, Platania CB, Forte S, Salomone S, Drago F, Bucolo C. MicroRNA target prediction in glaucoma. Prog Brain Res. 2015;220:217-40.).

In summary, there are still no reports on the use of tetrahedral DNA or miR-22 in the treatment of glaucoma, let alone the combined use of the two as optic neuroprotective drugs in the treatment of glaucoma. In order to overcome the difficulties in the treatment of glaucoma, it is urgent to further develop neuroprotective drugs that can effectively treat optic nerve diseases.

### Summary of the Invention

The object of the present invention is to provide a drug for the treatment of optic nerve diseases.

The present invention provides a complex for the treatment of optic nerve diseases, which is composed of the tetrahedral DNA and miR-22 according to the molar ratio of 1: (1 ~ 4).

The tetrahedral DNA of the present invention is a three-dimensional DNA nanostructure with a tetrahedral shape, which is formed by DNA sequence design, complementary pairing principle and automatic hybridization combination of each strand. In the present invention, four single-stranded DNAs have the nucleotide sequences as shown in SEQ ID NO. 1 ~ SEQ ID NO. 4. A complex of tetrahedral DNA and microRNA is formed by linking a microRNA toan end of the single strand of the tetrahedral DNA. In the present invention, the specific microRNA is miR-22.

Further, the tetrahedral DNA is formed by 4 single-stranded DNAs through complementary base pairing; the 4 single-stranded DNAs have the sequences selected one-to-one from the sequences as shown in SEQ ID NO. 1 ~ 4, respectively; wherein the end of one or two or three or each of the single-stranded DNAs is linked to miR-22; miR-22 has the sequence as shown in SEQ ID NO. 5.

Further, the above-mentioned miR-22 is linked to 1 ~ 4 single-stranded DNAs of the four single-stranded DNAs forming the tetrahedral DNA structure by a chemical bond or chemical bonds.

Further, there is a linker sequence between miR-22 and the single-stranded DNA(s). The linker sequence is a nucleotide sequence, preferably a deoxyribonucleotide sequence, more preferably -TTTTT-.which is a sequence of 5 consecutive thymine deoxynucleotides.

The present invention also provides a method for the preparation of the above-mentioned complex, wherein 4 single-stranded DNAs of the tetrahedral DNA are maintained at a temperature sufficient to denature them for more than 10 min, and then the temperature is lowered to 2 ~ 8°C, maintaining for more than 20 min; and one or more of the4 single-stranded DNAs as mentioned above is linked to miR-22.

Further, the 4 single strands of the tetrahedral DNA are maintained at 95°C for 10 min, and then the temperature is lowered to 4°C, maintaining for 20 min.

The present invention also provides the use of the above-mentioned complex in the preparation of a medicament for treating optic nerve disease. Further, the above-mentioned medicament for the treatment of optic nerve diseases is an optic neuroprotective drug; preferably, the optic nerve disease is associated with retinal ganglion cell injury, and/or retinal ganglion cell apoptosis.The drug can slow retinal ganglion cell injury, reduce retinal ganglion cell apoptosis and promote retinal ganglion cell survival; more preferably, the said retinal nerve disease is associated with brain-derived neurological factor (BNDF)-related signaling pathways, and the drug can promote the release of BDNF.

Further, the optic nerve disease is glaucoma; and in particular, primary open angle glaucoma.

The present invention also provides a pharmaceutical composition for the treatment of optic nerve diseases, which comprises the above-mentioned complex for the treatment of optic nerve diseases and pharmaceutically acceptable excipients.

The present invention also provides a method for the treatment and/or prevention of optic nerve diseases, comprising administering an effective amount of the complex of tetrahedral DNA and miR-22 of the present invention or the pharmaceutical composition of the present invention to a patient in need thereof. The optic nerve disease is preferably glaucoma.

The experimental results show that tFNA-miR22, a complex of tetrahedral DNA and miR-22 of the present invention, can effectively inhibit apoptosis of retinal ganglion cells induced by N-methyl-D-aspartate (NMDA) and promote the release of brain-derived neurophilic factor (BDNF), so as to play a good protective effect on retinal ganglion cells. The application of tFNA-miR22 for the preparation of an optic nerve protective drug will be helpful for the treatment of neurodegenerative optic nerve diseases including glaucoma, which has a very good application prospect.

Obviously, according to the above contents of the present invention, other various forms of modifications, replacements or alterations can be made in accordance with the general technical knowledge and conventional means in the field, without departing from the above basic technical ideas of the present invention.

The above contents of the present invention will be further described in detail by means of specific embodiments in the form of examples. However, it should not be construed as limiting the scope of the above-mentioned subject matter of the present invention to the following examples.. All technologies implemented based on the above contents of the invention belong to the scope of the invention.

### Description of the Drawings

Fig. 1 shows the schematic diagram of the synthesis of tetrahedral DNA and miR-22;
Fig. 2 shows the detection results of capillary electrophoresis;
Fig. 3 shows the detection results of PAGE electrophoresis of tFNA-miR22, tetrahedral DNA and its single-strands (1: S1, 2: S2, 3: S3, 4: S3-miR22, 5: S4, 6: tFNA, 7: tFNA-miR22);
Fig. 4 shows the images of tetrahedral DNA by transmission electron microscopy (a) and atomic force microscope (b), and detection results of Zeta potential and particle size of tetrahedral DNA (c-d);
Fig. 5 shows the images of tFNA-miR22 by transmission electron microscopy (a) and atomic force microscope (b), and detection results of Zeta potential and particle size of tFNA-miR22 (c-d);

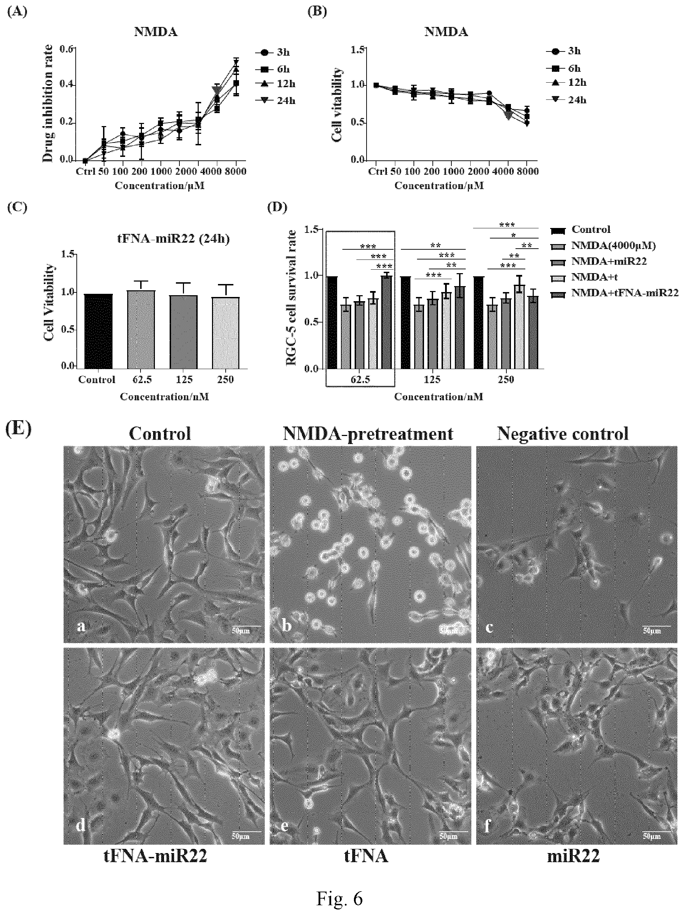

Fig. 6 shows the establishment of in vivo and in vitro modeling of optic nerve injury by NMDA and the detection results of cell activity of the NMDA-treated retinal ganglion cells with different concentrations of tFNA-miR22: A-B: CCK-8 activity detection and drug inhibition rates of the cells which were stimulated by different concentrations of NMDA for 1h and cultured in complete medium for 3, 6, 12, and 24 h; C: biosafety detection of tFNA-miR22; D: CCK-8 activity detection of cells which were treated with 4 nM NMDA and then treated with different concentrations of tFNA-miR22, tetrahedral DNA and single-stranded miR-22 for 24h; E: cell morphology in each group after the above treatment under ordinary light microscope. Data from A~ D refer to mean ± standard deviation (with sample size of each group >_ 3);

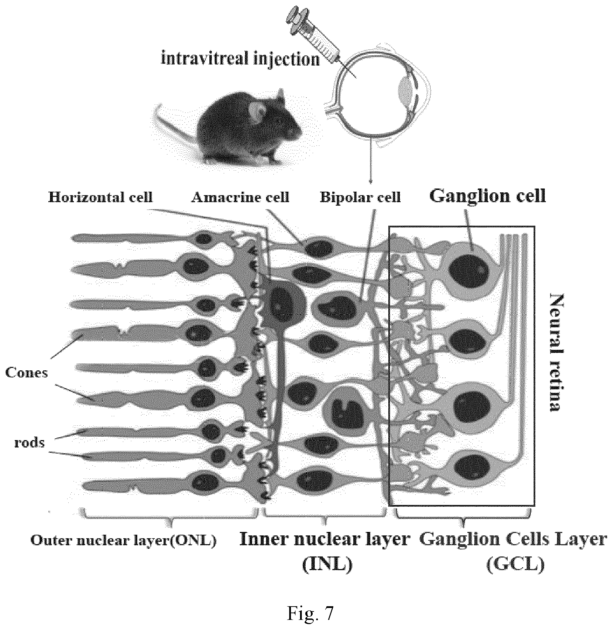

Fig. 7 shows a schematic diagram of the establishment of an in vivo model;
Fig. 8 shows the results of hematoxylin-eosin staining;
Fig. 9 shows the images of immunofluorescence staining of retinal flat-mounts and data analysis, and the data refer to mean ± standard deviation (with sample size of each group >_ 3);
Fig. 10 shows the results of the cell penetration rate of tFNA-miR22 and single-stranded miR-22 (Cy5 fluorescent-labeled) within 3, 6, 12 and 24 h detected by flow cytometry;
Fig. 11 shows the uptake results of tFNA-miR22 and single-stranded miR-22 (Cy5 fluorescent-labeled) for 6 h detected by immunofluorescence;
Fig. 12 shows the effect of 62.5 nM tFNA-miR22, tetrahedral DNA and single-stranded mi-R22 on cell cycle detected by flow cytometry and data analysis, the statistical data refer to mean ± standard deviation (with sample size of each group ≥ 3);
Fig. 13 shows the apoptosis of cells in each group detected by flow cytometry and statistical data analysis, the statistical data refer to mean ± standard deviation (with sample size of each group >_ 3).
Fig. 14 shows (A) the expression of apoptosis-related proteins detected by Western blot; (B) statistical analysis of anti-apoptotic protein Bcl-2; (C) statistical analysis of apoptotic protein Bax; (D) statistical analysis of apoptotic protein Caspase-3;
Fig. 15 shows the immunofluorescence staining of anti-apoptotic protein Bcl-2and statistical analysis;
Fig. 16 shows the immunofluorescence staining of apoptosis protein Bax and statistical analysis;
Fig. 17 shows the immunofluorescence staining of apoptotic protein Caspase-3 and statistical analysis;
Fig. 18 shows the relevant detection of TrKb-Creb-BDNF signaling pathway: A: expression level of TrkB/BDNF protein analyzed by Western blot (GAPDH as internal reference); B: relative expression level of TrkB protein; C: relative expression level of BDNF protein; D: expression level of Ntrk2 gene; E: expression level of BDNF gene;
Fig. 19 shows (A) expression of ERK1/2-CREB protein analyzed by Western blot. (B) relative expression levels of ERK1/2 and phosphorylated ERK1/2 protein; (C) relative expression levels of CREB and phosphorylated CREB proteins;
Fig. 20 shows the expression of TrkB selectively activated by tFNA-miR22 detected by immunofluorescence and statistical analysis;
Fig. 21 shows the expression of BDNF detected by immunofluorescence and statistical analysis;
Fig. 22 shows the expression of p-ERK1/2 detected by immunofluorescence and statistical analysis;
Fig. 23 shows the expression of p-CREB detected by immunofluorescence and statistical analysis;
Fig. 24 shows the expression results of TrkB protein and BDNF protein by immunohistochemical staining.

### Detailed Description of the Invention

The raw materials and equipment used in the present invention are known products and commercially available.

### Example 1. Synthesis of the complex of tFNA and miR-22 (tFNA-miR22)

Four DNA single strands, one of which was connected to miR22 at its end (S1, S2, S3-miR22, S4), were dissolved in TM Buffer (10 mM Tris-HCl, 50 Mm MgCl₂, pH=8.0) at a final concentration of 1000 nM for each of the four DNA single strands, fully mixed, rapidly heated to 95°C, maintaining for 10 min, and then rapidly cooled to 4°C, maintaining for more than 20 min, to obtain tFNA-miR22.

The sequences of the four single strands (5'→3') were as follows:
S1: 
ATTTATCACCCGCCATAGTAGACGTATCACCAGGCAGTTGAGACGAAC
ATTCCTAAGTCTGAA
 (SEQ ID NO.1)
S2: 
ACATGCGAGGGTCCAATACCGACGATTACAGCTTGCTACACGATTCAG
ACTTAGGAATGTTCG
(SEQ ID NO.2)
S3: 
ACTACTATGGCGGGTGATAAAACGTGTAGCAAGCTGTAATCGACGGGA
AGAGCATGCCCATCC
(SEQ ID NO.3)
S4: 
ACGGTATTGGACCCTCGCATGACTCAACTGCCTGGTGATACGAGGATG
GGCATGCTCTTCCCG
(SEQ ID NO.4)
   miR-22:
   AAGCUGCCAGUUGAAGAACUGU
(SEQ ID NO.5)
   S3-miR22-3p: 
AAGCUGCCAGUUGAAGAACUGU-TTTTT-ACTACTATGGCGGGTGATA
AAACGTGTAGCAAGCTGTAATCGACGGGAAGAGCATGCCCATCC
(SEQ ID NO.6)
 Wherein, the 5' end of S1 was optionally linked to a Cy5 fluorescent label group for tracing tFNA-22.

### 2. Identification

The DNA single-strands and synthetic tFNA-miR22 were detected by capillary electrophoresis and PAGE electrophoresis. The morphology of tFNA and tFNA-miR22 were detected by transmission electron microscopy. The zeta potential and particle size of tFNA and tFNA-miR22 were detected by dynamic light scattering.

### 3. Identification results

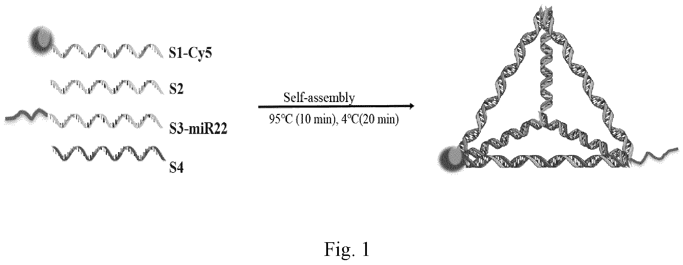

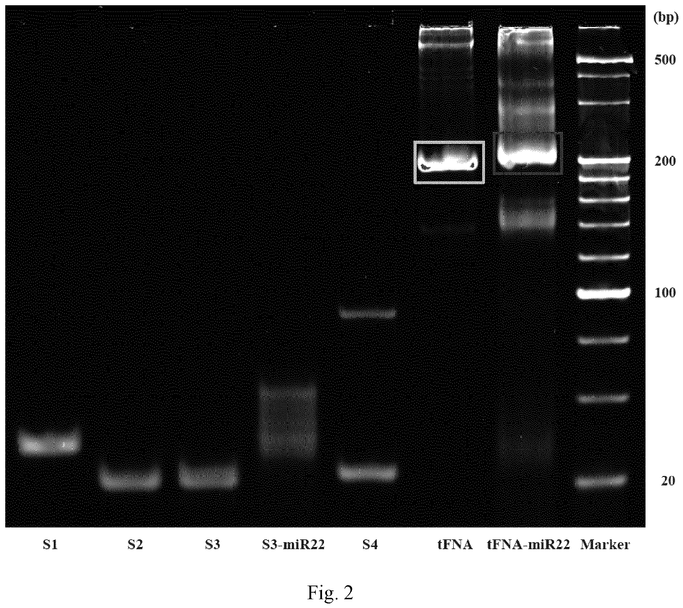

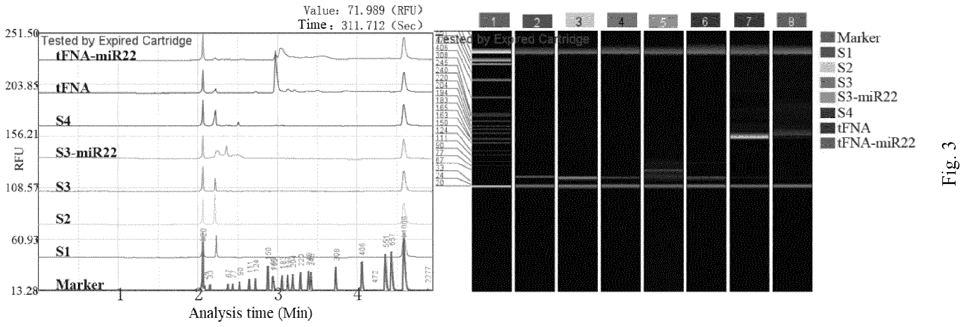

As shown in Figs. 1-3, the electrophoresis results showed that the molecular weight of tFNA-miR22 band was significantly higher than that of the single-stranded DNAs and the tetrahedral DNA, indicating that the single-stranded DNAs were assembled together.

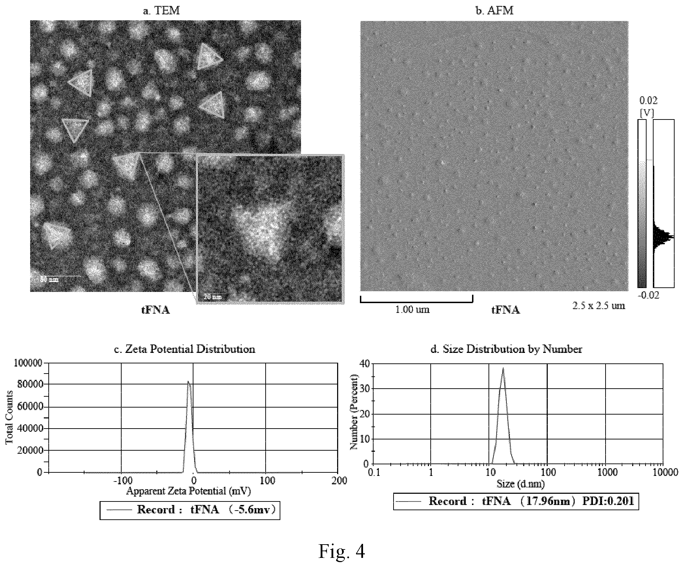

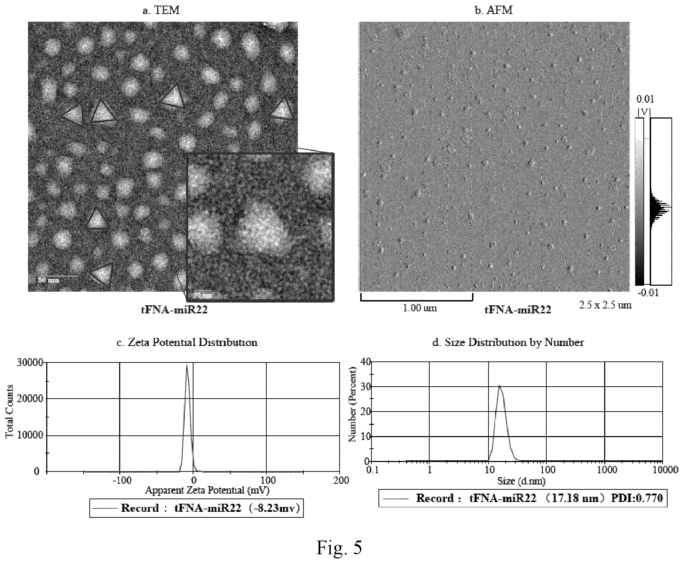

As shown in Figs. 4-5, the tetrahedral structure particles were detected by transmission electron microscopy. It was found by dynamic light scattering that the zeta potential of tFNA was 5.6 and the particle size was 17.96 nm; the zeta potential of tFNA-miR22 was 8.23 mV and the particle size was 17.18 nm, indicating that tFNA-miR22 was successfully synthesized and stable.

The beneficial effects of the present invention will be further described by way of experimental examples. The tFNA involved in the experimental examples was prepared by the method of Example 1.

### Experimental example 1. Uptake of tFNA-miR22 by injured retinal ganglion cells

### 1. Experimental methods

### 1.1 Test of the optimal modeling concentration (in vitro simulation of optic ganglion cell injury)

RGC-5 cells (a type of mouse retinal ganglion cells) were cultured in groups in 96-well plates with 1*10⁴ cells per well. Each group was treated with different concentrations of N-methyl-D-aspartate (NMDA) for 1h, and then cultured with complete medium for 24 h and then the cell activity was detected by CCK-8 assay. It was found that the drug inhibition rate of 4 mM NMDA was about 40%, so 4 mM was selected as the optimal modeling concentration (Fig. 6: A-B).

### 1.2 Test of the optimal anti-cell injury concentration of drug (cell proliferation experiment)

RGC-5 cells were cultured in groups in 96-well plates with 1*10⁴ cells per well. Each experimental group except the blank group was treated with 4 nM NMDA for 1 h, and then cultured for another 24 h with a culture medium containing 0 nM, 62.5 nM, 125 nM and 250 nM tFNA and tFNA-miR22 prepared in the example 1, as well as the single-stranded miR-22, respectively. Samples were taken and detected for cell activity by CCK-8 assay. It was found that tFNA at 62.5 nM had no obvious proliferative effect, while tFNA-miR22 at this concentration could significantly promote the proliferation of RGC-5 cells, moreover, the proliferation ratio of the cell viability treated with tFNA-miR22 compared to the cell viability of the NMDA control group was even higher than the sum of the proliferation ratios of the cell viability of miR22 or tFNA alone compared to the cell viability of the NMDA control group, indicating that the combination of miR22 and tFNA into tFNA-miR22 played a synergistic role in promoting the proliferation of NMDA-injured ganglion cells. Therefore, 62.5 nM was selected as the optimal drug concentration for this experiment. (Fig. 6: D).

### 1.3 Test of material uptake by injured cells

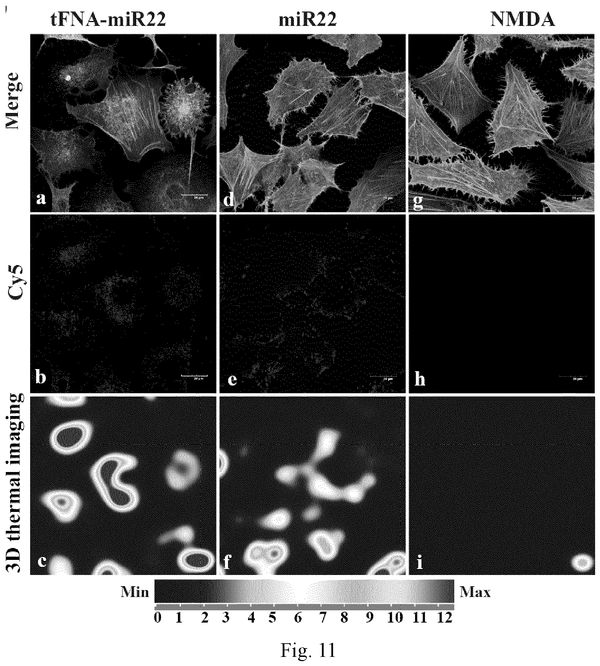

RGC-5 cells treated with 4 mM NMDA for 1h were grouped and then exposed to and treated with Cy5-labeled single-stranded miR-22 (62.5 nM) and tFNA-miR22 (62.5 nM) for 3 h, 6 h, 12 h, and 24 h, respectively, and compared with the injured group (i.e., untreated with tFNA and tFNA-miR22). All groups were washed 3 times with phosphate buffer and detected with flow cytometry. It was found that the fluorescence intensity of tFNA-miR22 reached its peak at 6 h (Fig. 11). Therefore, RGC-5 cells treated for 6 h by the above method were selected to prepare cell slides, and the uptake of single-stranded miR-22 and tFNA-miR22 was observed by immunofluorescence staining.

### 2. Results

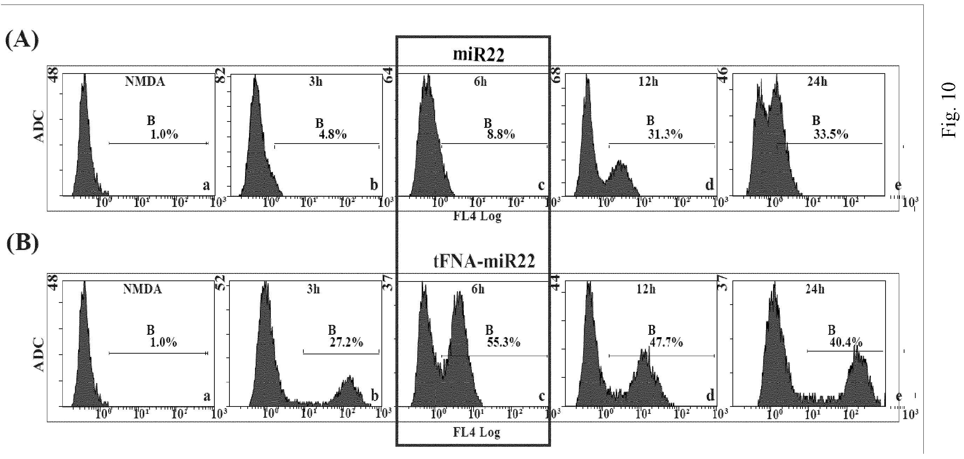

As shown in Figs. 10-11, the results of cell flow cytometry showed that within 24h, the fluorescence intensity of tFNA-miR22 reached a peak of 55.3% at 6 h, and gradually decreased to 40.4% with the increase of treatment time, while the fluorescence intensity of single-stranded miR-22 increased slowly with the treatment time, and reached a peak of 33.5% at 24 h. The results of immunofluorescence staining in Fig. 11 showed that tFNA-miR22 was widely accumulated in the cytoplasm and perinuclear of RGC-5 at 6h, while single-stranded miR-22 mainly adhered to the surface of cell membranes.

The above results indicate that tFNA-miR22 can be taken up more rapidly and efficiently by injured RGC-5 cells, while miR-22 that is not attached to tFNA is difficult to be taken up by RGC-5 cells.

### Experimental Example 2. tFNA-miR22 inhibits NMDA-induced cell injury

### 1. Experimental methods

RGC-5 cells were treated with 4 mM NMDA for 1 h, and then treated with 62.5 nM single-stranded miR-22, tFNA or tFNA-miR22 for 24 h, and detected as follows:
1) Cell morphology was observed by phase contrast microscope;
2) Cell cycle was detected by flow cytometry;
3) Cell apoptosis rate was detected by flow cytometry;
4) Expression of Bax, caspase-3 and Bcl-2 was detected by immunofluorescence and Western blot.

### 2. Results

1) Fig. 6C showed that tFNA-miR22 had no significant cytotoxicity, indicating that tFNA-miR22 has good biological safety.
2) Fig. 6E shows that compared with tFNA and single-stranded miR-22, tFNA-miR22 can significantly protect the morphology of retinal ganglion cells.

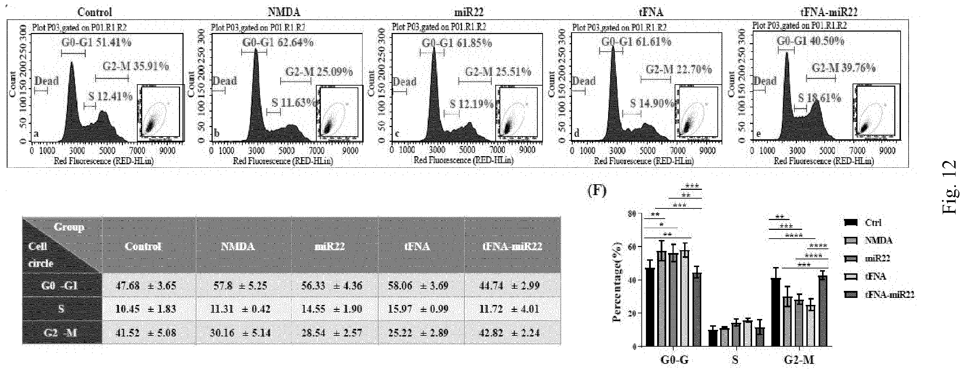

3) Fig. 12 shows that compared with tFNA and single-stranded miR-22, tFNA-miR22 can significantly promote cell self-renewal by regulating cell mitosis; and it can be clearly seen that the mitosis of the NMDA-treated cells was affected, and compared with the control group, the percentage of cells in G2-M phase in the NMDA group significantly decreased; and after the treatment with tFNA or miR22 alone, the percentage of cells in G2-M phase even further decreased. However, after the treatment with tFNA-miR22, the percentage of cells in G2-M phase significantly increased, even comparable to the control group without NMDA interference. It can be seen that the combination of tFNA and miR-22 had an opposite effect compared with use of them alone. They can synergize with each other to significantly promote cell mitosis and self-renewal.

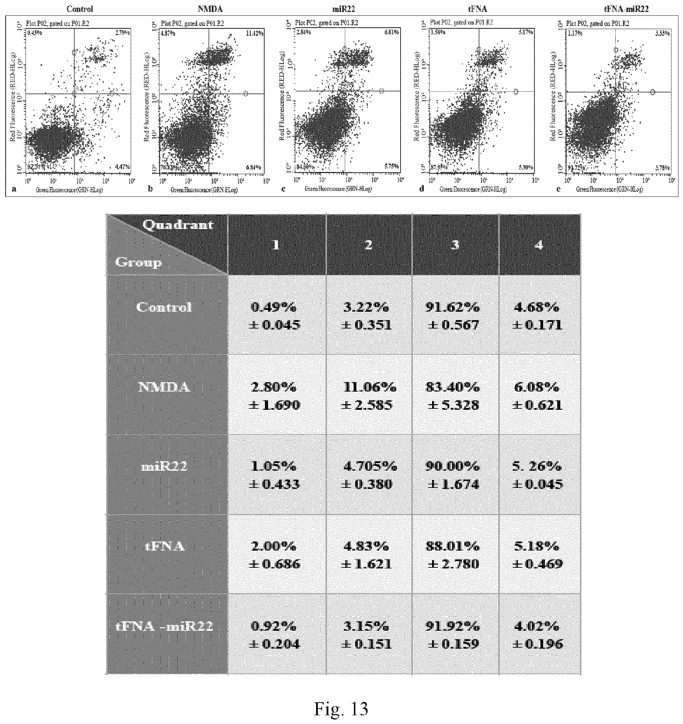

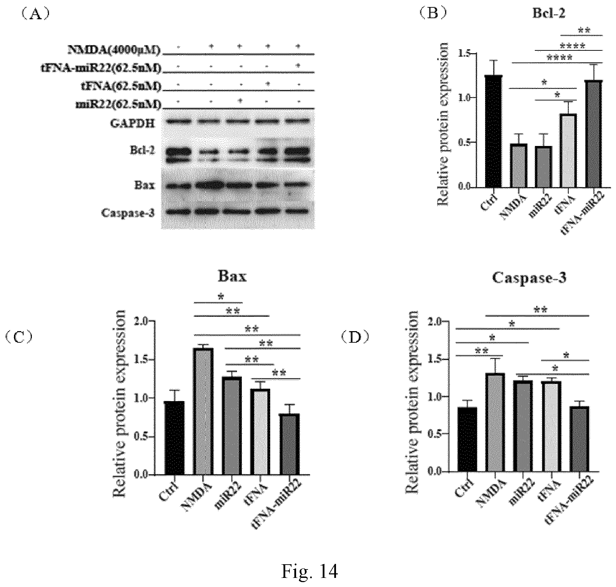

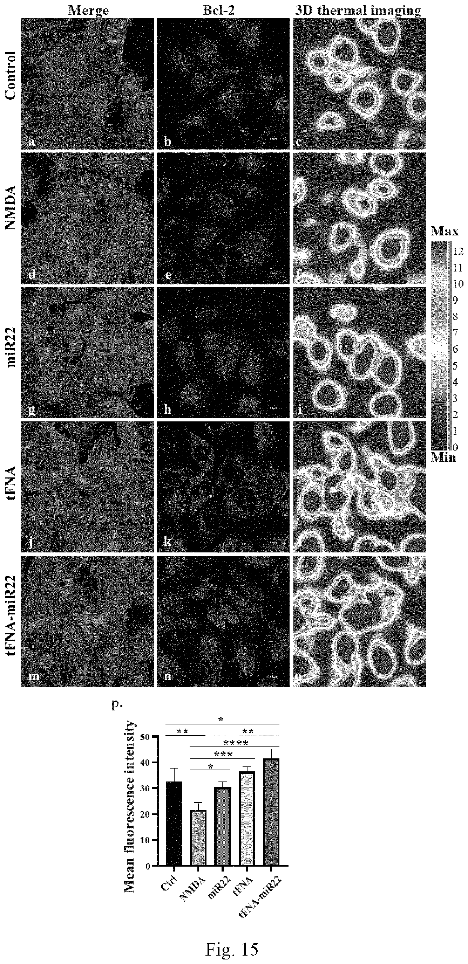

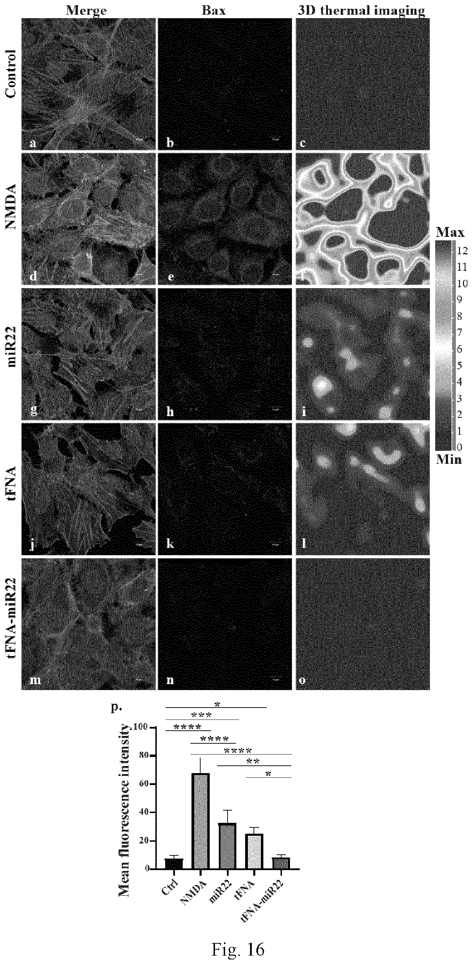

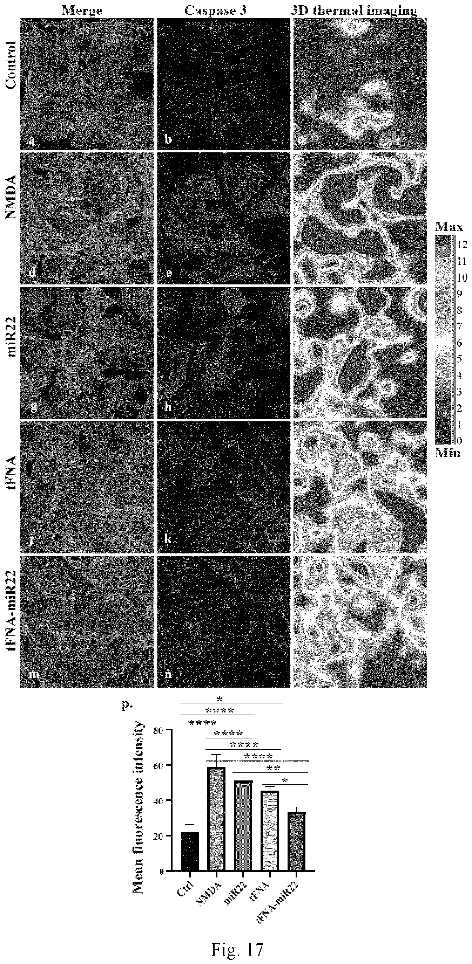

4) Fig. 13-17 showed that tFNA-miR22 can inhibit NMDA-induced apoptosis compared with tFNA or single-stranded miR-22, i.e.,tFNA-miR22 can reduce the NMDA-induced up-regulated expression level of apoptosis protein caspase-3 and Bax, and reduce the NMDA-induced down-regulated expression level of anti-apoptosis protein BCL-2.

The above results indicate that tFNA-miR22 has good biosafety and protective effect on retinal ganglion cells. tFNA-miR22 can regulate cell mitosis, promote cell self-renewal, and can reduce the expression of pro-apoptotic proteins caspase-3 and Bax by increasing the expression of anti-apoptosis protein BCL-2, thereby reducing the cell injury caused by NMDA, so as to further play a role in cell protection, and has a significantly better effect than tFNA or single-strand miR-22 alone.

### Experimental Example 3. Effect of tFNA-miR22 on TrkB/BDNF signaling pathway

### 1. Experimental methods

RGC-5 cells were treated according to the method of Experimental example 2, and detected as follows:
1) BDNF and Trkb proteins were detected by Western blot and immunofluorescence;
2) Expression levels of BDNF and Ntrk2 were detected by RT-PCR;
3) ERK1/2, p-ERK1/2, CREB and p-CREB proteins were detected by Western blot and immunofluorescence.

### 2. Results

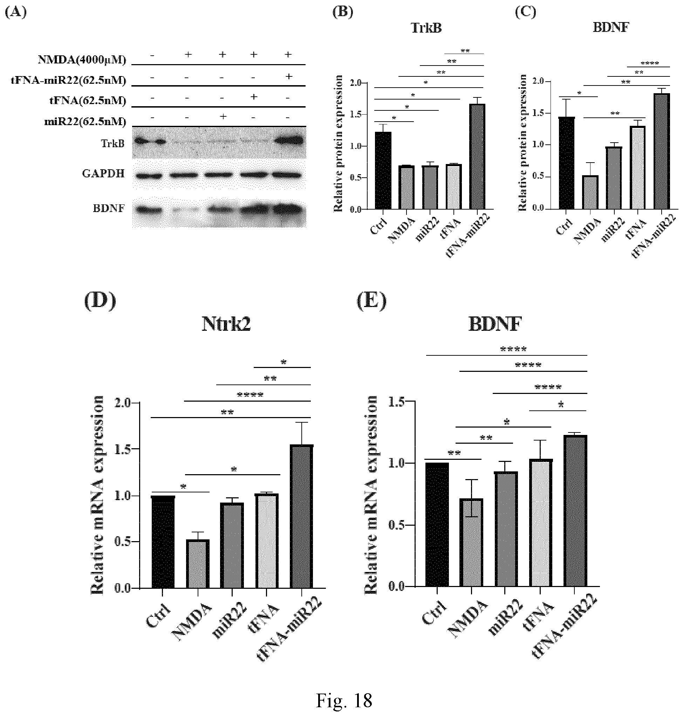

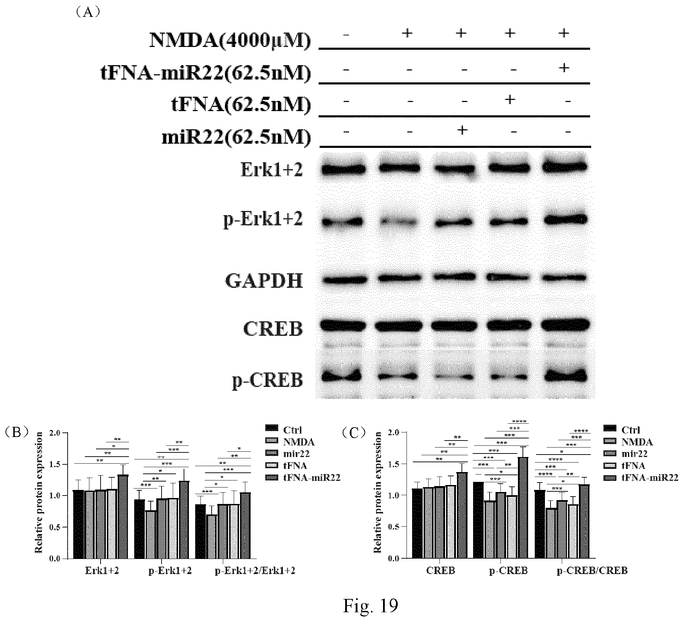

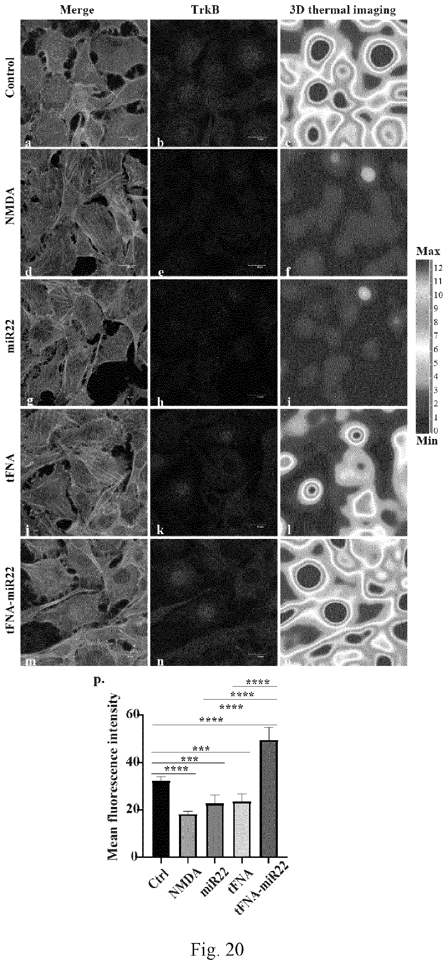

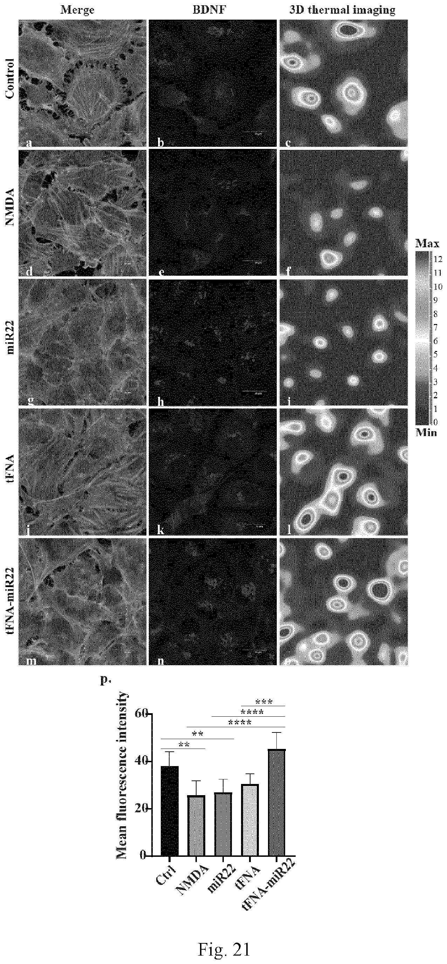

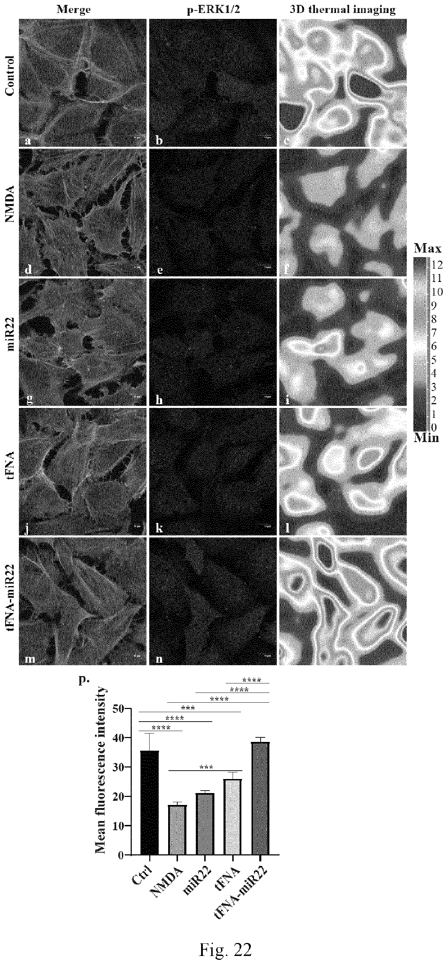

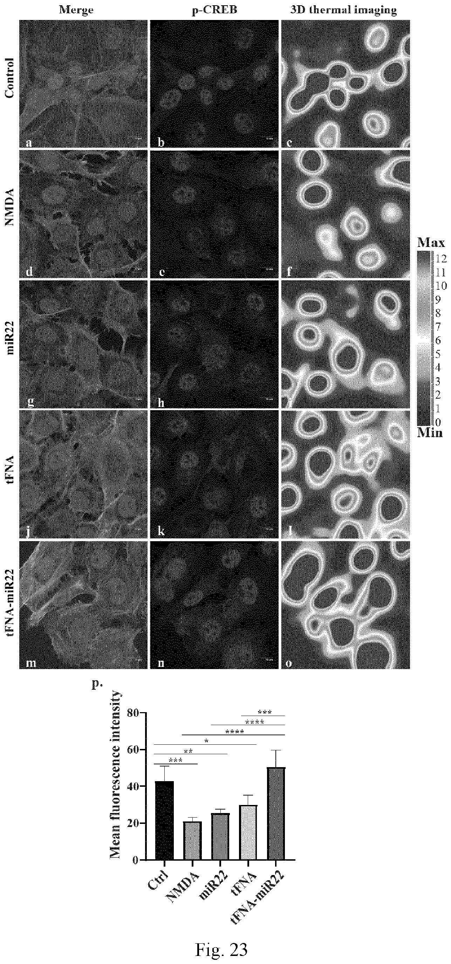

1) The detection of Western blot in Fig. 18A-C showed that the levels of BDNF and TrkB were significantly increased in tFNA-miR22-treated cells. Real-time quantitative PCR results in Fig. 18D-E showed that the gene expression of Ntrk2 and BDNF were significantly increased in tFNA-miR22-treated cells compared with the rest of the groups.
2) The detection of Western blot in Fig. 19 showed that the total proteins of ERK and CREB in tFNA-miR22-treated cells were increased to a certain extent, and could promote the phosphorylation of ERK1/2 and CREB.
3) The detection results of immunofluorescence of the above proteins shown in Figs. 20-23 were consistent with the detection results of Western blot.

The purpose of this experimental example is to further confirm the mechanism of tFNA-miR22 in producing protective effect on optic nerve.

Brain-derived growth factor (BDNF) is a powerful neuroprotective agent, especially for retinal ganglion cells. BDNF is one of the key neurotrophic factors in glaucoma. By binding to its receptor TrkB, BDNF may activate the extracellular signal-regulated kinase (ERK), which leads to the phosphorylation of cAMP response element-binding protein (CREB), thereby inducing the transcription of various genes associated with neuronal survival and promoting cell survival.

The above results indicate that tFNA-miR22 selectively activates TrkB, and by activating the downstream signaling pathway (ERK-CREB), promotes the release of BDNF to reduce cell injury and promote cell survival.

### Experimental example 4: Treatment of NMDA-induced optic nerve injury model mice with tFNA-miR22

### 1. Experimental methods:

Establishment of NMDA-induced optic nerve injury model
1) Selection and grouping of experimental animals: The experimental subjects were 6-week-old healthy male C57BL/6J mice, weighing 18-20 g. After examination, there was no obvious crooked neck, the cornea was transparent, the iris blood vessels were clear, the pupils were large and round, and they were sensitive to light reflection.. The experimental animals were randomly divided into five ABCDE groups by random number table method, which were blank control group, NMDA injury group, tFNA alone treatment group (62.5 nM), miR-22 alone treatment group and tFNA-miR22 treatment group (62.5 nM), respectively.
(2) Group treatment: After the mice were satisfactorily anesthetized, both eyes of the mice in each group were taken as experimental eyes and the eye surface was disinfected with 10% tincture of iodine. Under the surgical microscope, a 32G needle was punctured at 1mm from the temporal temporalis margin of the horn sclera, and then 2 µL drug was injected into the vitreous cavity with a 10 µL microsyringe. Group A: normal mice without surgery; Group B: injected with 2 µL of NMDA prepared in saline at a final concentration of 20 µM; Group C: injected with 1 µL of NMDA (20 µM) + 1 µL of tFNAs (62.5 nM); Group D: injected with 1 µL of NMDA (20 µM) + 1 µL of miR-22 (62.5 nM); Group E: injected with 1µL NMDA (20 µM) + 1 µL tFNAs-miR22 (62.5 nM). After the operation, erythromycin ophthalmic ointment was applied to the conjunctival sac. The animals were sacrificed 7 days after the operation to remove the eyeball with a section of the optic nerve retained.. The following morphological tests were carried out::
   A) Retinal histological changes were observed by HE staining;
   B) Immunofluorescence staining of whole retinal flat-mounts: RGCs counting;
   C) Expression of BDNF and Tkrb was observed by immunohistochemical IHC staining of routine retinal sections.

### 2. Results

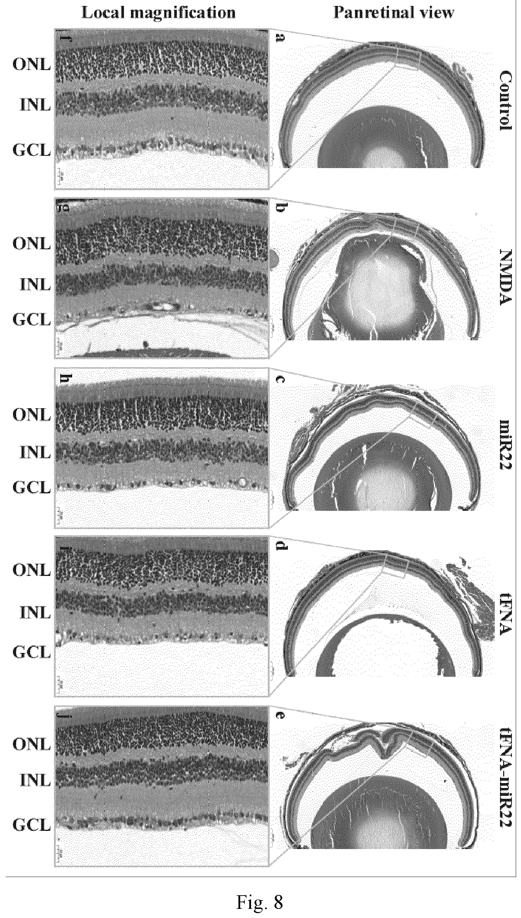

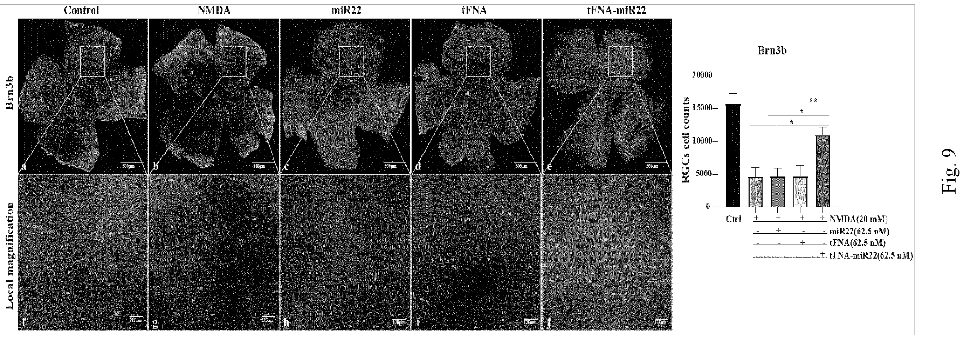

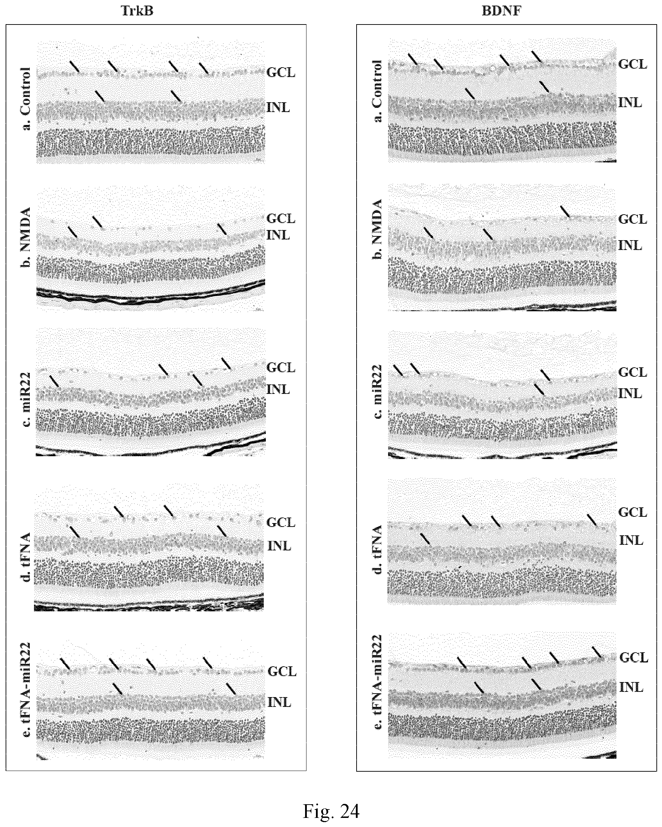

1) The results of HE staining in Fig. 8 showed that after tFNAs-miR22 treatment, the retinal thickness increased significantly and the number of ganglion cells increased significantly.
2) The immunofluorescence staining results of the flat-mounts are shown in Fig. 9. After tFNAs-miR22 treatment, the number of ganglion cells increased significantly, with statistical significance; the number of ganglion cells in tFNA-miR22 treatment group significantly increased compared with the NMDA control group, while the number of ganglion cells after treated with tFNA or miR22 alone was almost unchanged compared with the NMDA treatment group .
3) IHC staining results are shown in Fig. 24.: After tFNAs-miR22 treatment, the expressions of BDNF and Tkrb in the retina were significantly increased.

It can be concluded that the tFNA-miR22 group significantly increased the survival rate of optic ganglia cells compared with the other groups, indicating that the complex tFNA-miR22 of the present invention has an optic nerve protective effect, and can be used in the treatment of neurodegenerative optic nerve diseases including glaucoma; and has a significantly better effect than tFNA and miR-22 alone, indicating that the two have a synergistic effect.

In summary, the present invention provides a neuroprotective drug that can be used to treat neurodegenerative optic nerve diseases including glaucoma, which comprises tFNA-miR22 composed of a tetrahedral DNA and a miR-22 in a molar ratio of 1: (1-4). tFNA-miR22 can not only be effectively taken up by injured RGC-5 cells, but also effectively inhibit the apoptosis of retinal ganglion cells, and promote the release of brain-derived nerve factor (BDNF), thereby playing a good protective effect on retinal ganglion cells.

## Claims

1. A complex for treating optic nerve diseases, **characterized in that** it is composed of a tetrahedral DNA and a miR-22 in a molar ratio of 1: (1-4).

2. The complex of claim 1, **characterized in that** the tetrahedral DNA is formed from 4 single-stranded DNAs through complementary base pairing; the 4 single-stranded DNAs have the sequences as shown in SEQ ID NO. 1 ~ 4, respectively; the tetrahedral DNA is linked to the miR-22 at one or more single-strand end thereof, and the miR-22 has the sequence as shown in SEQ ID NO.5.

3. The complex of claim 1 or 2, **characterized in that** miR-22 is linked by a chemical bond to 1 ~ 4 of the 4 single-stranded DNAs forming the tetrahedral DNA structure.

4. The complex of claim 3, **characterized in that** there is a linker sequence between the miR-22 and the linked single-stranded DNA.

5. The complex of claim 4, **characterized in that** the linker sequence is -TTTTT-.

6. A preparation method of the complex of any one of claims 1 to 5, **characterized in that** the preparation method comprises putting the four single-stranded DNAs forming the DNA tetrahedron at a temperature sufficient to denature them for more than 10min, and then reducing the temperature to 2-8 ° C for more than 20min., wherein one or more of the 4 single-stranded DNAs are linked to the miR-22.

7. The preparation method of claim 6, **characterized in that** putting the four single-stranded DNAs forming the DNA tetrahedron at 95 ° C for 10min, and reducing the temperature to 4 ° C for 20min.

8. Use of the complex according to any one of claims 1 to 5 in the preparation of a drug for the treatment of optic nerve diseases.

9. The use of claim 8, **characterized in that** the drug for the treatment of optic nerve diseases is an optic nerve protective drug.

10. The use of claim 8, **characterized in that** the said optic nerve disease is selected from diseases associated with retinal ganglion cell injury and retinal ganglion cell apoptosis.

11. The use of claim 10, **characterized in that** the said optic nerve disease is associated with the regulation of brain-derived neurogenic factor-related signaling pathways.

12. The use of claim 8, **characterized in that** the said optic nerve disease is glaucoma.

13. A pharmaceutical composition for the treatment of optic nerve diseases, **characterized in that** the composition comprises the complex of any one of claims 1 to 4 and pharmaceutically acceptable excipients.

14. A method of treating optic nerve diseases, comprising administrating the complex of claim 1 or the pharmaceutical composition of claim 13 to a patient in need thereof.
